# EUROPEAN PATENT APPLICATION

(11) **EP 2 295 972 A1**
(43) Date of publication of application: **16.03.2011**
(21) Application number: 09169946.2
(22) Date of filing: 10.09.2009
(51) Int. Cl.: G01N 33/569, G01N 33/68, A61K 39/35

(54) **S. aureus allergen**

(71) Applicant: BIOMAY AG, 1090 Wien (AT)
(72) Inventor: Valenta, Rudolf, 2604, Theresienfeld (AT); Reginald, Kavita, 1150, Vienna (AT); Westritschnig, Kerstin, 1190, Vienna (AT); Linhart, Birgit, 3610, Weissenkirchen in der Wachau (AT); Stöcklinger, Angelika, 5020, Salzburg (AT); Thalhammer, Josef, 5112, Lamprechtshausen (AT); Hirschl, Alexander M., 1060, Vienna (AT); Werfel, Thomas, 30629 Hannover (DE); Greisenegger, Elli, 1060 Vienna (AT); Kopp, Tamara, 1180 Vienna (AT)
(74) Representative: Sonn & Partner Patentanwälte

(57) **Abstract**

The invention discloses the use of a protein with an amino acid sequence according to SEQ.ID.NO.1 or a naturally occurring fragment or variant thereof for identifying infections with S. aureus in a human sample.
More specifically, IgE molecules specific for SEQ.ID.NO.1 are detected in the sample of the patient. Many atopic dermatitis patients have IgE specific for SEQ.ID.NO.1.

## Description

The present invention relates to a novel allergen from Staphylococcus aureus and diagnostic and therapeutic uses thereof.

Atopic dermatitis (AD) is a chronic inflammatory skin disease which affects up to 20.5% of children and between 2.1 to 8.8% of adults. The clinical manifestations of AD vary and can range from dry skin and eczematous lesions to intense pruritus and lichenified flextures. It has been reported that about 80% of AD patients exhibit elevated levels of serum IgE, and the IgE levels are often correlated with disease severity. As AD is associated with other atopic diseases such as asthma and allergic rhinitis, patients with AD often have specific IgE antibodies and allergic symptoms to great variety of food and inhalant allergens.

Individuals suffering from AD show increased susceptibility to cutaneous bacterial, viral and fungal infections. The predominant skin infection in AD is caused by Staphylococcus aureus, which affects between 29-100% of patients. S. aureus is present at 100-1000 fold higher density (about 10⁵ cfu/mL) in the skin of AD patients compared to the skin of healthy individuals. Only 5-8% of healthy persons harbour S. aureus, and is usually concentrated in the mucosal cavities. Density and frequency of S. aureus colonization is significantly correlated with the severity of eczema. Furthermore, treatment of S. aureus skin infections with anti-staphylococcal antibiotics significantly reduces bacterial count and clinical severity of the disease.

Beginning from the early 1980s, several groups reported that specific IgE against S. aureus proteins could be detected in the serum of AD patients. However, not all AD patients produced IgE against S. aureus. Detectable anti S. aureus IgE titers were mostly observed in patients with moderate to severe AD. S. aureus superinfected AD patients showed higher total and specific anti-staphylococcal IgE levels compared to patients without superinfections. During the same period, several research groups reported that AD patients produced specific IgE against both cellular proteins and cell wall components of S. aureus.

Commonly, the standard clinical practice used for treating bacterial infection is by the administration of antibiotics. In the case of S. aureus, it has steadily acquired resistance to antibiotics, beginning with meticillin in 1961, followed by penicillin, and more recently vancomycin. Its resistance to multiple antibiotic classes has resulted in wide spread infection with this bacteria, leading to the development of vaccines against S. aureus. Several vaccines have already been generated, and are in clinical trials. Of these, two vaccines which entered the phase III trial, has both failed at this stage (Schaffer et al., 2008). It is likely that the generation of a successful S. aureus vaccine would need to consist of a mixture of antigens with different properties.

It is an object of the present invention to provide means for improving diagnosing and/or therapy strategies for patients having AD or being at risk of getting AD due to infection with S. aureus.

Therefore, the present provides the use of a protein with an amino acid sequence according to SEQ.ID.NO.1 or a naturally occurring fragment or variant thereof for identifying infections with S. aureus in a human sample.

The protein with an amino acid sequence according to SEQ.ID.NO.1 is a protein from S. aureus. This protein has been described as "2C2". The full length open reading frame of cDNA coding for the 2C2 protein variant according to SEQ.ID.NO.1 is 1698 base pairs (SEQ.ID:NO2). This cDNA translates to a 565 amino acid protein, having a theoretical molecular weight of 65.8 kDa, and pI of 8.72. 2C2 has 2 domains, a fibrinogen binding protein A domain (FbpA) matching amino acids 4 to 442 and a second domain termed DUF814 (domain of unknown function) matching amino acids 447 to 533.

Naturally occurring variants of this protein (which are commonly referred to herein by the term "2C2" have been identified as the result of genome wide search for virulence and resistance proteins in S. aureus strains (see Table 1). 2C2 does not share high amino acid sequence similarity to any known protein from virulent and clinically relevant bacteria. This safeguards a low cross-reactivity to known proteins which makes 2C2 a useful and specific marker for S. aureus infections. This protein has also allergenic activity. Furthermore, serum of patients with AD contain specific IgE molecules against 2C2, proving the usefulness of this protein as a diagnostic marker for AD. In addition, 2C2 is able to stimulate T cell proliferation in a dose dependent manner and to induce allergenic activity in human and mammals.

Since 2C2 is specific for S. aureus and unique, it is possible to detect S. aureus also in complex samples, such as human blood or tissue samples. 2C2 as used herein relates to the protein with SEQ.ID.NO.1 and all naturally occurring variants thereof, such as the ones defined in Table I. Amino acid exchanges in variants of SEQ.ID.NO.1 are therefore preferably located at positions 88, 102, 111, 169, 171, 211, 216, 244, 258, 289, 302, 441, 558 and 565. Presence of nucleic acid and amino acid sequences of naturally occurring variants can easily be detected and analysed in S. aureus containing isolates by standard methods, e.g. PCR and sequencing.

Naturally occurring fragments of 2C2 are also suitable for the purpose of the present invention. These fragments are sufficiently large (above 5 kDa, preferably above 10 kDa) to allow a suitable detection via usual detection methods, such as antibody binding, etc.. These fragments (such as a 17 kDa and a 22 kDa fragment (kDa as determined in SDS PAGE; corresponding to K439 to S565 (17 kDa) and K381 to S565 (22 kDa))) are stable (otherwise, they would not be detectable in natural isolates (e.g. from the skin or mucosa of human patients) by ordinary detection methods, such as ELISA); these fragments are therefore specifically suitable as marker peptides. "Naturally occurring" relates to any full length 2C2 polypeptide or fragment which can be detected (e.g. by a "state-of-the-art" ELISA using polyclonal anti-2C2-antibodies) in an S. aureus isolate or in a culture for recombinantly expressing 2C2 (in prokaryotic or eukaryotic host cells) or in an individual which has been infected with S. aureus.

The present invention relates to a method for analysing a sample for identifying infections with S. aureus wherein the presence or absence of 2C2 (i.e. a protein with an amino acid sequence according to SEQ.ID.NO.1 or a naturally occurring variant thereof) or a naturally occurring fragment thereof in the sample is tested.

Currently, bacteria (especially those to be identified in human clinical samples) are mainly identified by culture techniques which take long time. For example, detection of bacteria from blood cultures may take several days, which means that a patient could be already dead which means that therapy has to be initiated in a blind way. On the other hand, the importance of the correct diagnosis is evident because it allows to select the suitable antibiotic treatment which may safe the life in case of infections and help especially in AD patients because patients suffering from S. aureus skin colonization benefit from antibiotic therapy.

With the present invention determining the presence or absence of S. aureus in such a sample can be performed within a very short time by standard test methods, such as ELISA or PCR without the need for culturing bacteria in the sample. Moreover, an assay based on the 2C2 protein, its fragments or on nucleic acids encoding these polypeptides, is also free from the bias of the requirement of viable bacteria for culturing technique (which could bring false negative results in the case that all bacteria in the samples are not propagatable anymore or false positive results if the culture is contaminated).

Based on the DNA and amino acid sequence according to SEQ.ID.NO.2 and SEQ.ID.NO.1, the present invention also provides e.g. a PCR assay for identification of S. aureus infections or a sandwich ELISA assay.

In a suitable ELISA according to the present invention, the 2C2 protein (or its fragments) may be caught out of a biological sample with a 2C2-specific antibody and detected with a second antibody directed against a different epitope of 2C2 or an antibody being specific for the previous binding event.

Presence of 2C2 or a fragment thereof (or presence of nucleic acid encoding such polypeptides) in a sample of an individual is indicative for an acute infection with S. aureus in (or on) this individual. Accordingly, human samples are preferred samples which are tested with respect to presence or absence of 2C2 or a naturally occurring fragment thereof.

Detection of 2C2 nucleic acid is possible by standard nucleic acid tests such as PCR (e.g. Asymmetric PCR, Linear-After-The-Exponential-PCR (LATE-PCR), Helicase-dependent amplification, Hot-start PCR, Ligation-mediated PCR, Miniprimer PCR, Ligation-dependent Probe Amplification (MLPA), Multiplex-PCR, Nested PCR, Quantitative PCR (Q-PCR), RT-PCR, Solid Phase PCR, Touchdown PCR (Step-down PCR), or PAN-AC).

The present invention therefore also provides a kit for determining the presence or absence of S. aureus nucleic acid in a biological sample, comprising: (a) a primer set comprising at least two synthetic nucleic acid sequences for amplifying at least one nucleic acid sequence encoding 2C2 or parts thereof, wherein at least of one of the synthetic nucleic acid sequences in the kit is selected from the group consisting of: (a) a suitable primer pair, the primers being a nucleic acid sequence comprising 10-30 consecutive nucleotides of at least one of the following: (i) the 2C2 gene according to SEQ.ID.NO.2 or the complementary sequence thereof; or (ii) from the 5' or 3' noncoding region of the 2C2 gene which is available from the S. aureus genome projects (Gill et al., Kuroda et al., Ohta et al., Diep et al., Baba et al., Holden et al., Herron-Olson et al.) (here, regions extending up to 500, preferably up to 200, especially up to 100, nucleotides into the 5'- or 3-direction are specifically preferred under (B)) or complementary sequences thereof; said primers being usable for polymerising a nucleotide sequence located between the primers on the 2C2 gene or its 5'- or 3'-region (said polymerizable 2C2 nucleotide sequence being PCR detectable and preferably up to 1000 bp, more preferred up to 500 bp, even more preferred up to 300bp in length; on the other hand, the primers may also be selected to polymerise (with PCR) the whole 2C2 DNA); and (b) suitable reagents for a polymerase chain reaction (PCR). Preferably, the kit further comprises: (c) instructions for use and (d) optionally, a positive and/or negative control for determining the presence or absence of S. aureus, especially a control nucleic acid encoding 2C2 or a fragment thereof. Examples of suitable PCR reagents include PCR reaction buffer, Mg²⁺ (e.g., MgCl₂), dNTPs, DNA polymerases (such as reverse transcriptases and thermostable DNA polymerases (e.g., Taq-related DNA polymerases and Pfu-related DNA polymerases)), RNase, PCR reaction enhancers or inhibitors, PCR reaction monitoring agents (e.g., double-stranded DNA dye (such as SYBR^{™} Green), TaqMan^{™} probes, molecular beacons, and Scorpions^{™}), and PCR-grade water.

The primers described herein are particularly useful in a polymerase chain reaction (PCR) assay. PCR is a practical system for in vitro amplification of a DNA base sequence. For example, a PCR assay may use a heat-stable polymerase and two about 10 to 30 base primers: one complementary to the (+)-strand at one end of the sequence to be amplified, and the other complementary to the (-)-strand at the other end. Because the newly-synthesized DNA strands can subsequently serve as additional templates for the same primer sequences, successive rounds of primer annealing, strand elongation, and dissociation may produce rapid and highly-specific amplification of the desired sequence. PCR also may be used to detect the existence of a defined sequence in a DNA sample.

By way of example, a typical PCR assay might start with two synthetic oligonucleotide primers which are specifically and complementarily binding to two regions of the target DNA or its complementary strand, respectively, encoding 2C2 or its 5'- and/or 5' region (one for each strand) that is to be amplified. These may be added to the target DNA (that need not be pure) in the presence of excess deoxynucleotides (dNTPs) and a thermostable DNA polymerase (e.g., Taq polymerase). In a series (typically 20-40) of temperature cycles, the target DNA may be repeatedly denatured (about 80-100°C, e.g. 90°C), annealed to the primers (typically at 40-65°C), and a daughter strand may be extended from the primers (typically at 65-80°C, e.g. 72°C). As the daughter strands themselves act as templates for subsequent cycles, DNA fragments matching both primers are amplified exponentially, rather than linearly. The target DNA need be neither pure nor abundant; thus, PCR is specifically suitable in the clinical diagnostics according to the present invention.

Other preferred tests for identifying 2C2 nucleotides in a sample include the hybridisation of nucleic acids of this sample with 2C2-specific probes (probes that specifically recognise a target nucleic acid encoding 2C2 or its flanking regions) in hybridization tubes wherein the probe is preferably linked to a solid surface, such as a magnetic bead or a microarray.

These tests may preferably employ suitable labels, such as biotin, fluorescent molecules, radioactive molecules, chromogenic substrates, chemiluminescence markers, and the like. The methods for biotinylating nucleic acids are well known in the art, as are methods for introducing fluorescent molecules and radioactive molecules into oligonucleotides and nucleotides. Detection methods are well known for fluorescent, radioactive, chemiluminescent, chromogenic labels, as well as other commonly used labels. Briefly, chemiluminescence can be identified and quantitated most directly by their emission wavelengths and intensity. When biotin is employed, it is detected by avidin, streptavidin or the like, which is conjugated to a detectable marker, such as an enzyme (e.g. horseradish peroxidase). Steptavidin binds with high affinity to biotin, unbound streptavidin is washed away, and the presence of horseradish peroxidase enzyme is then detected using a luminescence-emission substrate in the presence of peroxide and appropriate buffers.

As said above, the fast and accurate diagnosis of a S. aureus infection is very important particularly in hospitalized patients and patients who have a suppressed immune system e.g. patients which have undergone or are in the course of chemotherapy etc..

Presence of antibodies being specific for 2C2 (or its fragments) is indicative of a previous infection with S. aureus.

Therefore, the present invention also relates to a method for detecting antibody molecules (especially IgE molecules) being specific for 2C2 (a protein with an amino acid sequence according to SEQ.ID.NO.1 or naturally occurring variants thereof) or antibody (especially IgE) binding fragments thereof in an antibody containing sample, wherein the sample is contacted with 2C2 (a protein with an amino acid sequence according to SEQ.ID.NO.1 or naturally occurring variants thereof) or antibody binding fragments thereof and whereafter it is analysed whether any of the antibody molecules have bound to 2C2 (a protein with an amino acid sequence according to SEQ.ID.NO.1 or naturally occurring variants thereof) or antibody binding fragments thereof. As already mentioned, this method according to the present invention is preferably used for the detection of IgE molecules (or antigen binding parts of IgE), however, also other 2C2 binding molecules can be detected and isolated with this method, specifically other antibody molecules can be detected according to the present invention, especially IgGs, such as IgG1, IgG2a and IgG4, e.g. in order to analyse the specificities of the S. aureus immune reaction in individual patients (e.g. for analysing whether a Th1 response has been induced after vaccination with 2C2). With the present method it is also possible to produce preparations containing 2C2 binding antibodies. This method is specifically suitable to prepare anti-2C2-IgE preparations or preparations of 2C2 specific monoclonal antibodies (which can be prepared by standard techniques using 2C2 or its naturally occurring fragments). On the other hand, also natural or synthetic binding partners can be identified and isolated by using this set-up, especially natural receptors or low molecular weight (e.g. below 2000 Da, below 1000 Da or below 500 Da) 2C2 binding molecules.

According to a preferred embodiment of this method, 2C2 (a protein with an amino acid sequence according to SEQ.ID.NO.1 or naturally occurring variants thereof) or antibody (especially IgE) binding fragments thereof is provided in an immobilised form on a solid carrier and then contacted with the antibody (especially IgE) containing sample. When the sample is contacted with the solid carrier, antibody (especially IgE) molecules being specific for 2C2 can, if present in the sample, bind to the immobilised polypeptides and then be detected. The contact conditions can easily be optimised, depending on the nature of the samples and the scale of the samples as well as on the nature of the assay (e.g. microarray detection allows shorter contact times than large scale ELISAs; for a qualitative assay (yes/no) shorter times are possible than for a reproducible quantitative assay for which a maximum number of possible binding events has to be established).

Preferably, the solid surface is separated from the sample before the (qualitative and/or quantitative) detection of the binding event. Therefore, in a preferred embodiment of the method according to the present invention the antibody (especially IgE) containing sample is separated from this solid surface after the contact, whereafter the analysis whether any of the antibody (especially IgE) molecules have bound to the protein with an amino acid sequence according to SEQ.ID.NO.1 or the antibody (especially IgE) binding fragment or variant thereof is performed on the solid surface in a form separated from the sample.

The methods according to the present invention allow the detection of an acute infection with S. aureus as well as a previous infection which resulted in an antibody production of the immune system of the infected individual against 2C2. Since S. aureus is one of the leading pathogens in hospitals, acute infections are preferably to be detected in samples of human patients in hospitals who had become infected e.g. due to their immunosuppresive state. On the other hand, the connection of anti-2C2 antibodies with AD according to the present invention allows the diagnosis of AD in patients which are suspected to have AD. This can either indicate a more specific or more pronounced treatment regimen for AD. In the treatment of AD it is very useful to know early whether there is a S. aureus infection, because then an appropriate antibiotic treatment can be applied. On the other hand, the methods according to the present invention also allow a proper subgrouping of AD patients. Accordingly, the present invention is preferably used to identify AD patients which have S. aureus colonization, as such patients can be given specific treatment. Additionally, the approximate 10% of AD patients not having S. aureus superinfection would not be unnecessarily given additional medication which they do not require.

Therefore, according to a preferred embodiment of the present method the sample is from a patient having or suspected to have (e.g. being at risk) atopic dermatitis (AD). The method according to the present invention is therefore highly appropriate for the diagnosis of atopic dermatitis in a human patient.

Typical formats of the diagnostic methods according to the present invention for which 2C2 or its antibody binding fragments or anti-2C2 antibodies can be used include radio-allergensorbent test (RAST), paper radioimmunosorbent test (PRIST), enzyme linked immunosorbent assay (ELISA), radioimmunoassays (RIA), immuno-radiometric assays (IRMA), luminescence immunoassays (LIA), histamine release assays and IgE immunoblots.

IgE binding activity of 2C2 and fragments thereof can be determined by, for example, an enzyme linked immunosorbent assay (ELISA) using, for example, sera obtained from an individual (i.e. an allergic individual) that has been previously exposed to 2C2 (e.g. by a previous infection with S. aureus). Briefly, 2C2 or a fragment thereof to be tested is coated onto wells of a microtiter plate. After washing and blocking the wells, antibody solution consisting of the serum or plasma of an individual who has been exposed to S. aureus before (or who has been immunised with 2C2) is incubated in the wells. The plasma could be depleted of IgG before incubation. However, the depletion is not necessary if a specific anti IgE-antibody is used. A labelled secondary antibody is added to the wells and incubated. The amount of IgE binding is then quantified and compared to the amount of IgE bound by a purified 2C2 or a purified fragment thereof.

Alternatively, the binding activity of a 2C2 fragment can be determined by Western blot analysis. For example, a fragment to be tested is run on a polyacrylamide gel using SDS-PAGE. The fragment is then transferred to nitrocellulose and subsequently incubated with sera from an allergic subject. After incubation with the labelled secondary antibody, the amount of IgE bound is then determined and quantified. Another assay which can be used to determine IgE binding activity of a 2C2 fragment is a competition ELISA assay. Briefly, an IgE antibody pool is generated by combining plasma or serum from individuals that have been shown by direct ELISA to have IgE reactive with 2C2. This pool is used in ELISA competition assays to compare IgE binding to 2C2 to the fragment tested.

The samples to be tested according to the present invention are preferably samples taken from the human skin or mucosa or of human blood, plasma, serum or lymph (the latter mainly for detecting antibodies against 2C2; these may also be detected in other biological samples, such as tears, nasal or bronchial secretions or liquor). Specifically the 2C2 protein or its naturally occurring fragments can, however, also be detected in other samples, such as human or animal tissue, faeces or urine samples, food samples, surface swabbing samples, etc.. Specifically food analysis is - besides the clinical diagnostic - interesting, because S. aureus intoxications are a major problem (e.g. vomitting or diarrhoea due to food). Identifying the cause for such intoxications (is it S. aureus or another microorganism, such as Salmonella, etc.?) is an important issue which has to be addressed by a food sample analysis which can include a method according to the present invention.

According to another aspect, the present invention relates to naturally occurring fragments of 2C2 in isolated form. 2C2 liberates smaller protein fragments which are stable and can be detected in samples. Preferred fragments according to the present invention are IgE binding fragments of 2C2 which are detected and isolated as described herein.

Such IgE binding fragments are specifically important in connection with AD diagnosis and vaccination, but also for other allergy patients.

The present invention also provides a new allergen, i.e. the 2C2 protein according to the present invention. This protein is first disclosed and provided in isolated form with the present invention. Although it was contained as open reading frame in S. aureus genome databases, its function has not been recognised, and in particular it has not been recognised as allergen and not provided in individualised and isolated form, e.g. in a suitable solution to be used as allergen in preparatory, diagnostic or scientific methods, and without any function to make it industrially applicable.

Specifically preferred fragments according to the present invention are a fragment having an apparent molecular weight in SDS page electrophoresis of 17 kDa and a fragment having an apparent molecular weight in SDS page electrophoresis of 22 kDa. When 2C2 is recombinantly produced in E. coli these two fragments are formed immediately during the expression process and appear stable to allow a proper detection. These fragments show specific binding affinity to 2C2 specific antibodies (especially IgEs) from human patients.

According to another embodiment, the present invention relates to a pharmaceutical composition comprising a protein with an amino acid sequence according to SEQ.ID.NO.1 or a naturally occurring fragment or variant thereof and a pharmaceutically acceptable carrier. 2C2 protein or a naturally occurring fragment thereof can be finalised with the pharmaceutically acceptable carrier in an appropriate form which allows administration to an individual, especially a human patient.

Pharmaceutically acceptable carriers preferably used are physiological saline, vegetable oils, mineral oil, aqueous sodium caroboxymethyl cellulose or aqueous polyvinylpyrrolidone; however, also sterile water can be used. The pharmaceutically useable formulations of the present invention may further comprise at least one pharmaceutical acceptable adjuvant or excipient. Preferred adjuvants include surface active substances, e.g., hexadecylamine, octadecylamine, octadecyl amino acid esters, lysolecithin, dimethyldioctadecylammonium bromide, methoxyhexadecylgylcerol, and pluronic polyols; polyamines, e.g., pyran, dextran-sulfate, poly IC, carbopol; peptides, e.g., muramyl dipeptide, dimethylglycine, tuftsin; oil emulsions; and mineral gels, e.g., aluminum hydroxide, aluminum phosphate, etc. and immune stimulating complexes. The adjuvant may be, for example, alum or a composition containing a vegetable oil, isomannide monooleate and aluminum mono-stearate. Other preferred adjuvants include microparticles or beads of biocompatible matrix materials.

The molecules of the present invention may be incorporated into microparticles or microcapsules to prolong the exposure of the antigenic material to the individual and hence protect said individual against infection for long periods of time. The immunogen may also be incorporated into liposomes or conjugated to polysaccharides and/or other polymers for use in a vaccine formulation. The present invention therefore also relates to a vaccine comprising a protein with an amino acid sequence according to SEQ.ID.NO.1 or a naturally occurring fragment or variant thereof and a pharmaceutically acceptable carrier. 2C2 and its naturally occurring fragments have proven to be allergenic and contain functional T-cell epitopes. The fact that 2C2 induces T cell proliferation also in normal persons and IgG antibodies shows that this protein can be used in general also for non-atopic persons. Moreover, the polypeptides according to the present invention are soluble and immunogenic. The vaccine according to the present invention preferably comprises further antigens, especially further antigens (epitopes) of S. aureus.

Typically, such vaccines are prepared as injectables: either as liquid solutions or suspensions, solid forms suitable for solution in, or suspension in, liquid prior to injection may also be prepared. The vaccine may be administered to a patient by any convenient route, such as subcutaneously, intraperitoneally, intramuscularly, intradermally, intravenously, orally, intranasally or intramammarily, in the presence of a physiologically acceptable diluent. 2C2 or immunogenic fragments thereof may be administered in a single dose or in a plurality of doses. The vaccine of the present invention may be stored under refrigeration or in frozen or lyophilized form. The vaccine is administered to an individual in an amount effective to elicit a protective immune response as compared to a control. The effective amount will vary, e.g., with the age and size and may be readily determined by the practitioner skilled in the art. Suitable regimes for initial administration and booster shots will also be variable, but may be typified by an initial administration followed by subsequent inoculations or other administrations.

With the disclosure according to the present invention, purified anti-2C2 antibody preparations can be provided. Raw materials containing such antibodies (e.g. human blood or lymph or antibody containing derivatives thereof) can be affinity-purified using 2C2 or antibody-binding fragments thereof. These polypeptides can e.g. be recombinantly produced (which is preferred) or extracted from S. aureus cultures and bound to a solid surface (e.g. a chromatographic material) to form an affinity column. After affinity purification, a preparation containing isolated anti-2C2 antibodies is obtained wherein all or at least the major antibody species is anti-2C2. Therefore, the present invention also provides a preparation containing isolated antibodies, especially IgEs and/or IgGs, against a protein with an amino acid sequence according to SEQ.ID.NO.1 or a naturally occurring fragment or variant thereof. 2C2 specific monoclonal antibodies can be prepared by standard techniques using 2C2 or its naturally occurring fragments. The monoclonal antibodies of the present invention can be prepared using classical cloning and cell fusion techniques. The immunogen (antigen) of interest (2C2 or one or more of its naturally occurring fragments) is typically administered (e.g. intraperitoneal injection) to wild type or inbred mice (e.g. BALB/c) or transgenic mice which produce desired antibodies, rats, rabbits or other animal species which can produce native or human antibodies. The immunogen can be administered alone, or mixed with adjuvant, or expressed from a vector (VEE replicon vector, vaccinia), or as DNA, or as a fusion protein to induce an immune response. Fusion proteins comprise the peptide against which an immune response is desired coupled to carrier proteins, such as β-galactosidase, glutathione S-transferase, keyhole limpet hemocyanin (KLH), and bovine serum albumin, to name a few. In these cases, the peptides serve as haptens with the carrier proteins. After the animal is boosted, for example, two or more times, the spleen is removed and splenocytes are extracted and fused with myeloma cells using the well-known processes of Köhler and Milstein and Harlow and Lane. The resulting hybrid cells are then cloned in the conventional manner, e.g. using limiting dilution, and the resulting clones, which produce the desired monoclonal antibodies, cultured. The antibodies can then be purified using the methods described herein, e.g. by (preparative) affinity chromatography to 2C2 columns, and optionally finalised to a pharmaceutical preparation.

These antibody preparations according to the present invention can specifically be used for detection of S. aureus or other diagnostic uses, especially in connection with S. aureus infections or AD. The antibodies according to the present invention can also be used for treating S. aureus infections (e.g. in the form of a passive vaccine).

According to another aspect, the present invention relates to a nucleic acid encoding a protein with an amino acid sequence according to SEQ.ID.NO.1 (SEQ.ID.NO.2) or a naturally occurring fragment or variant thereof, said nucleic acid being free of other open reading frames for proteins being naturally encoded adjacently to SEQ.ID.NO.2 in S. aureus. The present invention provides the 2C2 gene in isolated (and therefore useable) form. This means that the nucleic acid according to the present invention is free from its naturally present (in the S. aureus genome (see e.g. Gill et al., Kuroda et al., Ohta et al., Diep et al., Baba et al., Holden et al., Herron-Olson et al.)) adjacent (5' and/or 3') genes. This nucleic acid molecule according to the present invention can be used to produce recombinant 2C2 protein or fragments (in suitable prokaryotic (E.coli, B. subtilis, S. typhimurium, etc.) or eukaryotic (yeast, plant, animal (e.g. CHO, BHK, 293 cells) etc.) host cells) or to design suitable primers for PCR detection or probes for microarray detection.

Preferably, the nucleic acid according to the present invention is embedded in a suitable expression vector, such as a plasmid or a virus, which are well available for the skilled person in the art.

This aspect of the present invention also includes fragments of the 2C2 gene or its adjacent 5' and 3' regions, e.g. DNA molecules for use as PCR primers. These are usually about 10 to 30 nucleotides in length. Specific nucleic probes may be longer, e.g. 30 to 3000 bp, preferably 40 to 1000 bp, even more preferred 50 to 500 bp. These nucleic acids may also be immobilised on solid surfaces, e.g. on a microarray chip or on other PCR or sequencing surfaces and used for the methods according to the present invention.

The present invention is further illustrated by the following examples and the drawing figures, yet without being restricted thereto.

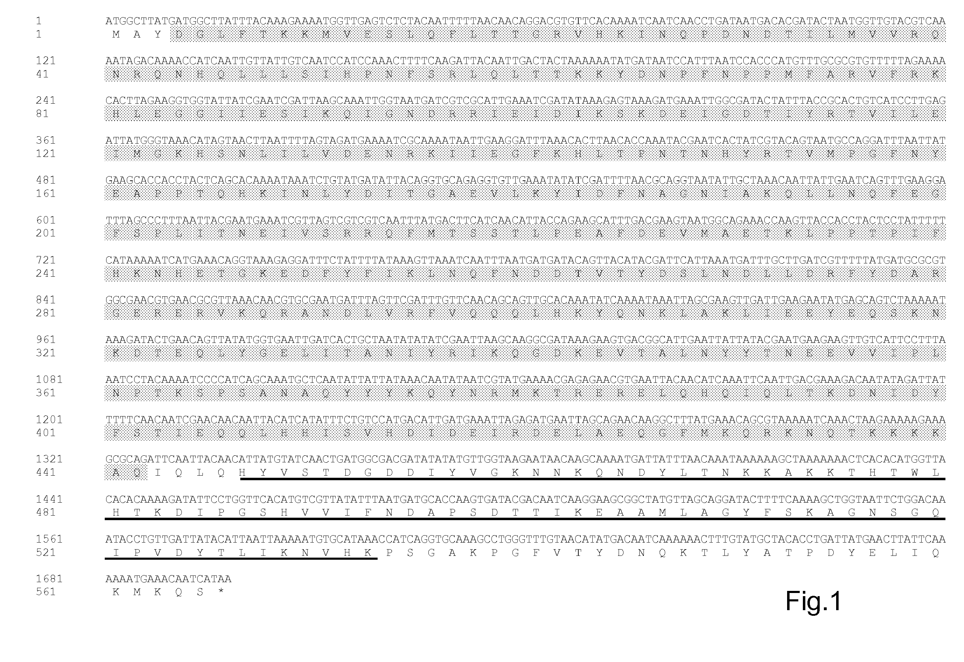

Fig.1 shows the nucleotide and deduced amino acid sequence of 2C2: Numbers on the left denote the positions of the nucleotides and the deduced amino acids. The TAA stop codon is indicated by (*). The 2C2 protein contains two domains, domain FbpA (shaded area) and domain DUF814 (underlined sequences).

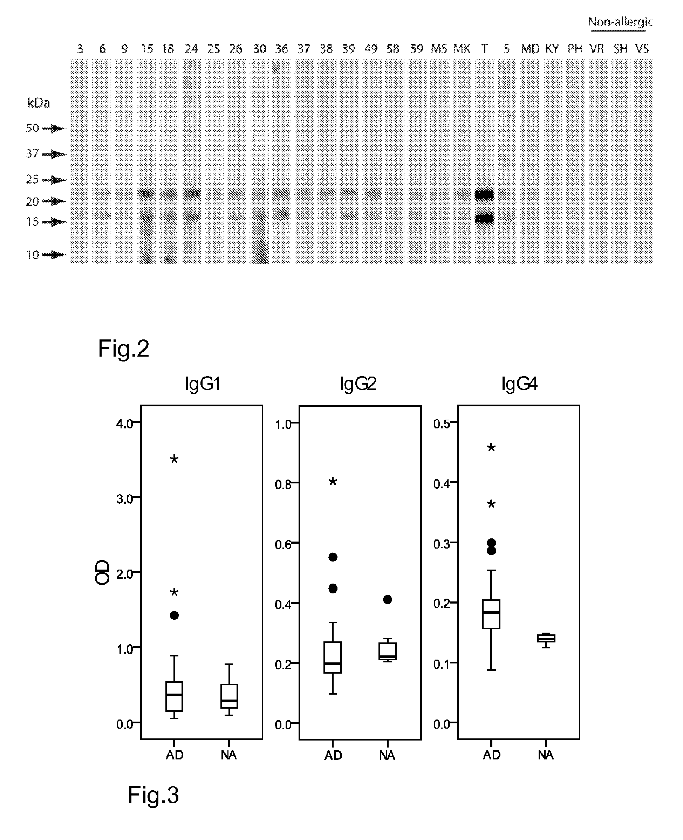

Fig.2 shows anti-2C2 IgE antibody reactivity of AD patients to nitrocellulose-blotted 2C2: Recombinant 2C2 protein was separated on SDS-PAGE and blotted onto nitrocellulose membranes. These membranes were probed with sera from AD patients, and non-allergic controls and amount of IgE binding was measured by autoradiography. Immunoblots of selected AD patients and non-allergic individuals are shown. Molecular weights (kDa) are indicated in the left margin.
Fig.3 shows levels of IgG1, IgG2 and IgG4 antibody subclasses in atopic dermatitis (AD) and non-allergic (NA) patients as measured by ELISA: Box plots represent the immunoglobulin levels in 37 AD and 8 NA patients measured in duplicates. Open circles represent outliers, while triangles represent extreme values.

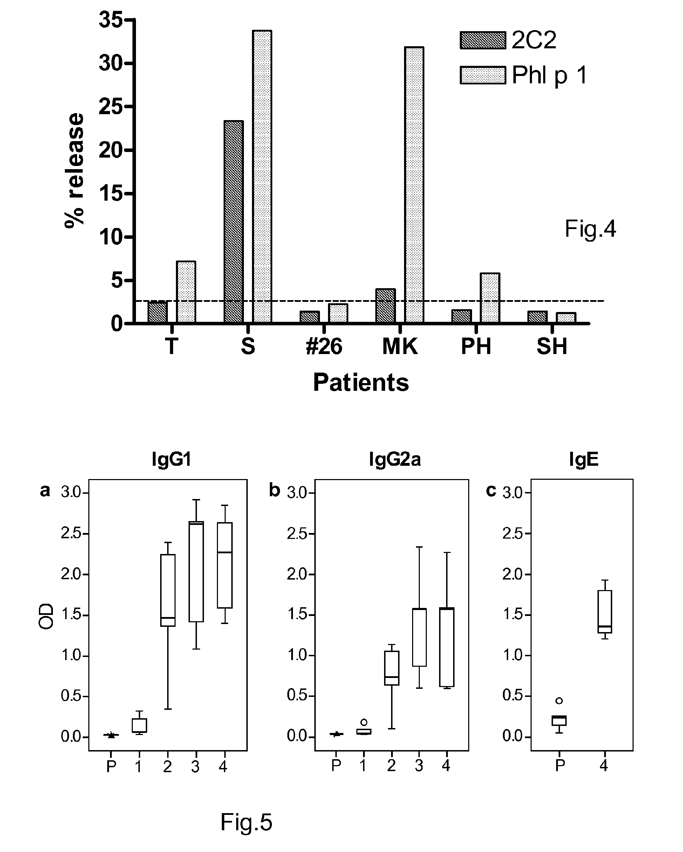

Fig.4 shows the humanized RBL assay: RBL cells transfected with cDNA coding for FcεRI were incubated with serum and 2C2 or Phl p 1. Upon IgE crosslinking, the humanized RBL cells release pre-formed mediators, and the amount of mediator release was recorded. The dotted line represents the baseline value.
Fig.5 shows levels of immunoglobulin production in mice immunized with 2C2: Mice (n=5) were immunized with 2C2 in monthly intervals. Sera from mice were collected at different time points (P, pre-immune sera; 1, one month after immunization; 2, two months after immunization, etc.) and the levels of a) IgG1, b) IgG2a, c) IgE antibodies were measured by ELISA. The levels of immunoglobulin are displayed as box-and-whiskers plot. Open circles represent outliers, while triangles represent extreme values.

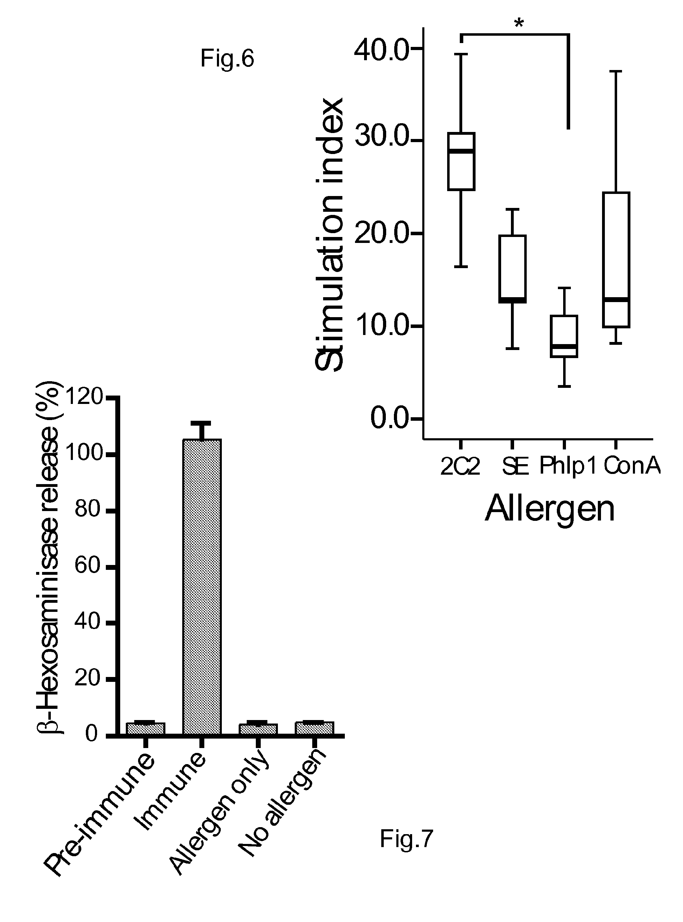

Fig.6 shows lymphocytes proliferation of mice spleen cells after immunization with 2C2: Mice spleen lymphocytes were stimulated with recombinant 2C2, SE, Phl p 1, or Con A. Lymphocyte proliferation was determined at day seven by [³H]thymidine incorporation and are expressed as stimulation indices. Triplicate measurements of proliferations were represented as box and whiskers plot.
Fig.7 shows the murine RBL assay: β-hexosaminidase release of rat basophilic leukaemia (RBL) cells sensitized with pre-immune or immune murine anti sera against recombinant 2C2. As controls, RBL cells were also stimulated with allergen or medium (no allergen) alone. Bars shown represent means and standard deviations of triplicate measurements.

### Examples:

### Materials and Methods

Selection of atopic dermatitis patients with specific IgE to S. aureus protein extracts:
For the preparation of S. aureus protein extract, overnight culture of the bacteria were harvested by centrifugation, washed twice with phosphate buffered saline (PBS) and homogenized using Ultra Turrax (IKA Labortechnik, Germany) and glass beads. The bacterial extract was then separated on a 12.5% preparative SDS-PAGE (Laemmli et al., 1970), and transferred onto nitrocellulose membranes (Schleicher & Schuell, Dassel, Germany) by electroblotting (Towbin et al., 1979). The membranes cut into 5 mm strips, and blocked with buffer A (50mM sodium phosphate buffer, pH 7.4, containing 0.5% w/v bovine serum albumin, 0.5% v/v Tween 20, and 0.05% w/v NaN3) for 2 h. Strips were then probed with serum from AD patients or healthy individuals at 4°C overnight. After thorough washing, bound IgE was detected by incubating the strips with ¹²⁵I-labeled anti-human IgE antibodies (Pharmacia, Uppsala, Sweden) diluted 1:10 in buffer A, overnight at room temperature and visualized by autoradiography.

Immunoscreening of the genomic S. aureus library with serum from atopic dermatitis patients:
A Lambda Zap II vector containing an S. aureus library strain RN 450 (Stratagene, La Jolla, CA, USA) was used to transform E. coli cells (XL1-Blue MRF' host strain). These plaques were then transferred to nitrocellulose membranes pre-treated with 10 mM isopropyl-1-thio-L-D-galactopyranoside (IPTG) (Stratagene) to induce protein expression. The membranes were then washed thrice in buffer A followed by overnight incubation with sera from AD at 4°C. The membranes were washed thoroughly, and IgE binding colonies were identified using ¹²⁵I-labelled anti IgE antibodies (Pharmacia, Uppsala, Sweden) diluted 1:15 in buffer A, and visualized by autoradiography. Positive plaques were cored and stored in 500 µL SM buffer (0.1M NaCl, 8.0 mM MgSO₄.7H₂O, 50 mM Tris-HCl) supplemented with 10 µL chloroform.

DNA Sequencing of IgE reactive clones:
DNA from IgE binding clones were isolated, and digested with Kpn I and Sac I to allow for directional cloning of the fragments in plasmid pUC18. The ligated product was transformed into E. coli XL-1 blue. Positive clones were identified by DNA restriction analysis, and double stranded DNA sequencing was performed using λgt 11 forward and reverse primers (Clontech). The cDNA and deduced amino acid sequences obtained were then compared with the entries in Genbank using the BLAST-X algorithm.

Expression and purification of recombinant 2C2 protein in E. coli:
The cDNA encoding the 2C2 protein was ligated to the pET23d (Novagen, Madison, WI, USA). The DNA sequence of the construct was confirmed by sequence analysis. The ligated vector was then transformed in E. coli BL21(DE3) (Stratagene, East Kew, Australia), and grown in LB-medium containing 100 mg/L ampicillin at 37°C. Protein expression was induced for 4 h by the addition of 1 mM isopropyl-β-thiogalactopyranoside when the OD₆₀₀ of the culture reached 0.5. Cells were harvested by centrifugation at 4000×g for 15 min at 4°C, and lysed by Ultra Turrax (IKA Labortechnik, Germany) in binding buffer (100 mM NaH₂PO₄, 10 mM Tris.Cl and 8M urea, pH 8.0). The hexahistidine recombinant protein was incubated with Ni-NTA resin (QIAGEN, Hilden, Germany) equilibrated for 1 h. The protein resin slurry was then loaded on columns, and washed twice with wash buffer B (100 mM NaH₂PO₄, 10 mM Tris.Cl and 8M urea, pH 6.3) followed by 2 washes with wash buffer C (50 mM NaH₂PO₄, 300 mM NaCl, 20 mM imidazole, pH 8.0) followed by elution with 50 mM NaH₂PO₄, 300 mM NaCl, 250 mM imidazole, pH 8.0. The eluted protein was then dialysed against distilled water, and the final protein concentration was determined by Micro-BCA Protein Assay Kit (Pierce, Rockford, IL, USA) according to the manufacturer's instructions.

IgE immunoblotting of 2C2:
Recombinant 2C2 protein was boiled for 5 min with sodium dodecyl sulfate (SDS) sample buffer containing 5% v/v β-mercaptoethanol (Laemmli et al., 1970). Approximately 3.5 µg of r2C2 per lane was separated on a 12.5% preparative SDS-PAGE (Laemmli et al., 1970). A protein molecular weight marker (Precision Plus Protein Kaleidoscope prestained standards, Bio-Rad, USA) was used as a standard. The separated proteins were then transferred onto nitrocellulose membranes by electroblotting (Towbin et al., 1979), and immunoblotting was performed as described in the previous section.

Animals:
Female 5 - 8 week old BALB/c mice were purchased from Charles River Laboratories and kept under specific pathogen free conditions. All experiments were approved by the local review board of the Medical University of Vienna and were performed in accordance with national and international guidelines of laboratory animal care.

Immunization of BALB/c mice:
Groups of mice (n=5) were sensitized via the subcutaneous route with 10 µg of r2C2, adsorbed to Al(OH)₃ (Alu-Gel-S; SERVA Electrophoresis) as previously described (Linhart et al., 2007). Mice were immunized at monthly intervals. Blood samples were taken from the tail veins and serum was stored at -20°C until analysis.

ELISA experiments:
The amount of 2C2-specific antibodies in the sera human patients or immunized mice were measured using ELISA. Microtitre plates (Nunc) were coated with 5 µg/mL of r2C2. Wells were blocked with buffer A for 2 h at room temperature. For the measurement of Ig subtypes in humans, human sera were diluted at 1/100 and incubated overnight at 4°C. Bound Abs were detected with monoclonal mouse anti-human IgG1, IgG2 or IgE Abs (BD Pharmingen) diluted 1/1000 and a HRP-coupled sheep anti-mouse antiserum (Amersham Biosciences) diluted 1/1000. For the detection of murine immunoglobulins, murine sera were diluted at 1/500 for IgG1, 1/100 for IgG2a, and 1/20 for IgE, bound Abs were detected with monoclonal rat anti-mouse IgM, IgG1, IgA and IgG2a Abs (BD Pharmingen) diluted 1/500 and a HRP-coupled goat anti-rat antiserum (Amersham Biosciences) diluted 1/2000.

Lymphocyte proliferation assays:
Heparinised blood from AD or control individuals were diluted at a ratio of 1:2 with PBS and peripheral blood mononuclear cell (PBMC) were isolated using Ficoll-Paque gradient centrifugation. PBMCs were resuspended in Ultraculture media (Bio Whittaker, Walkersville, MD, USA) supplemented with 2 mM glutamine, 50 mM β-mercaptoethanol and 0.1 mg/mL gentamycin. Cells were cultured in sterile 96 well plates (Nunc) at 2x10⁵ cells/well and stimulated with 2C2 (1.0 µg/well), Phl p 1 (1.0 µg/well), SE (10.0 µg/well), IL-2 (positive control) or medium (negative control) at 37°C with 5% CO₂. After 6 days, 0.5 µCi of ³H-thymidine was added to each well, and cells incubated for 16 h. The amount of ³H-thymidine incorporation was measured by scintillation counting and reported as stimulation index (SI), calculated as the ratio of the mean proliferation after antigen stimulation over medium control values. For mice lymphoproliferations, spleens of mice immunized with 2C2 were removed under aseptic conditions (at day 100 after the first immunization) and homogenized. After the lysis of erythrocytes, cells were washed and resuspended in complete medium (RPMI 1640, 10% FCS, 0.1 mg/mL gentamicin, and 2 mM glutamine). Single cell suspensions were plated into 96-well round-bottom plates (Nunc, Roskilde, Denmark) at a concentration of 2 x 10⁵ cells/well (200 µL) in triplicates and stimulated with Con A (0.5 µg/well), r2C2 (1.0 µg/well), SE (10.0 µg/well), rPhl p 1 (1.0 µg/well, Biomay, Vienna, Austria) or medium for six days. The amount of lymphocyte proliferation was measured as described above.

Rat basophil leukemia (RBL) and humanized RBL assays:
RBL-2H3 cells were cultivated in 96-well tissue culture plates (4 x 10⁴ cells/well) for 24 h at 37°C with 5% CO₂. Passive sensitization was performed by incubation with 1/10 diluted murine sera for 2 h. Cells were washed twice with Tyrode's buffer (137 mM NaCl, 2.7 mM KCl, 0.5 mM MgCl₂, 1.8 mM CaCl₂, 0.4 mM NaH₂PO₄, 5.6 mM D-glucose, 12 mM NaHCO₃, 10 mM HEPES, and 0.1% w/v BSA, pH 7.2) to remove unbound antibodies. Degranulation of RBL cells was induced by the addition of 0.3 µg/ml r2C2 and the amount of β-hexosaminidase released was analyzed after 30 min. Results were expressed as percentages of total β-hexosaminidase released after the addition of 1% Triton X-100 and represents the mean of triplicate determinations. For the humanized RBL assay, RBL cells transfected with cDNA coding for the human high-affinity IgE receptor chain, FcεRI was used. Cells (1 x 10⁵/well) were passively sensitization with sera from AD patients or healthy individuals at various dilutions (1:10, 1:30, 1:100 and 1:300) overnight in a 37°C, 5% CO₂ incubator. Cell were washed in Tyrodes buffer and degranulation was induced by the addition of 0.3 µg/mL 2C2, and the amount of β-hexosaminidase release was measured after 1 h. The amount of β-hexosaminidase was measured by mixing 50 µL of cell supernatant with assay solution (0.16 mM 4-methyl umbelliferyl-N-acetyl-D-glucosaminide in 0.1M citric acid, pH 4.5). The reaction was stopped by the addition of 100 µl glycine buffer, pH 10.7 and amount of fluorescence was measured at exication and emission wavelengths of 360 nm and 465 nm respectively.

Statistical analysis of the data:
Correlation between different parameters was tested by Mann-Whitmey U tests using SPSS^{®} software.

### Results:

Genomic library of S. aureus and screening of IgE reactive clones:
An immuno-screen of a λgt11 genomic library of S. aureus with serum IgE from six AD patients revealed the presence of 11 IgE-reactive clones. Phage DNA of these clones were isolated, and subjected to restriction analysis using Eco R I and Kpn I/Sac I and DNA sequencing. Based on the insert sizes after the restriction analysis, the clone with the biggest insert was also that with full length open reading frame, and so this clone (termed 2C2) was selected for further study.

DNA and amino acid sequence of 2C2:
The full length open reading frame of cDNA coding for an IgE reactive protein, 2C2, consists of 1698 base pairs. This cDNA translated to a 565 amino acids, having a theoretical molecular weight of 65.8 kDa, and pI of 8.72. By a search on the Pfam database (http://pfam.sanger.ac.uk/), 2C2 was shown to contain two domains, the fibrinogen binding protein A domain (FbpA) matching amino acids 4 to 442 and a second domain termed DUF814 (domain of unknown function) matching amino acids 447 to 533 (Figure 1). There were eight entries in the Genbank of amino acid sequences from S. aureus which shared more than 98% sequence identity with 2C2 (Table I).

**Table I. Entries of amino acid sequence in the Genbank which bear high similarities to 2C2**

| **No.** | **Access ion number** | **% identity** | **Changes in amino acid** | **Reference paper** |
|---|---|---|---|---|
| 1 | YP_186083 | 99 | L171P | Gill *et. al.*, 2005 |
| 2 | NP_371732 | 99 | L171P, S565L | Kuroda et. al., 2001; Ohta *et. al.*, 2004 |
| 3 | YP_493798 | 99 | L171P, M216I, H302Y | Diep *et. al.*, 2006 |
| 4 | YP_001246642 | 99 | L171 P, R289H, L558H | Unpublished |
| 5 | NP_645908 | 99 | 9E88K, D102E, L171P | Baba *et. al.,* 2002 |
| 6 | YP_040595 | 99 | I169M, L171 P, S211 N, L558H | Holden *et. al.,* 2004 |
| 7 | ZP_04018173 | 99 | D111N, I169M, L171P, S211N, L558H | Unpublished |
| 8 | YP_416554 | 98 | I169M, L171P, S211 N, H244N, Q258H, A441 E, L558H | Herron-Olson *et. al.,* 2007 |

The proteins listed in Table I were identified as the result of genome wide search for virulence and resistance proteins in S. aureus strains by several research groups. The next question was whether other pathogenic bacteria expressed proteins which were similar by amino acid sequence to 2C2. The amino acid sequence similarities between 2C2 and its homologous proteins from five other clinically relevant bacteria were tabulated (Table II).

**Table II. Percent pairwise sequence similarity between amino acid sequences of six fibronectin binding proteins**

| | **Similarity (%)** | | | | | |
|---|---|---|---|---|---|---|
| | **Staa** | **Step** | **Lism** | **Clod** | **Fusn** | **Borb** |
| **Staa** | 100.0 | 39.3 | 42.7 | 38.6 | 28.0 | 24.4 |
| **Step** | | 100.0 | 44.5 | 34.4 | 27.8 | 21.5 |
| **Lism** | | | 100.0 | 40.4 | 26.6 | 23.2 |
| **Clod** | | | | 100.0 | 30.0 | 24.3 |
| **Fusn** | | | | | 100.0 | 19.1 |
| **Borb** | | | | | | 100.0 |

| | | | | | | |
|---|---|---|---|---|---|---|
| *^{a}Staphylococcus aureus* (Staa), *Streptococcus pneumoniae* (Step), *Listeria monocytogenes* (Lism), *Clostridia difficile* (Clod), *Fusobacterium nucleatum*(Fusn), *Borrelis burgdorferi* (Borb) | | | | | | |

2C2 did not share high amino acid sequence similarity to any known protein from virulent and clinically relevant bacteria.

Expression and purification of 2C2 in E. coli:
The recombinant 2C2 was expressed as a hexahistidine protein in the E. coli expression system using IPTG induction. The proteins were purified using the nickel-NTA columns, and refolded on the column to obtain soluble protein. It was observed that this protein, which has a theoretical molecular weight of 65.8 kDa, liberates into many bands of smaller proteins molecular weights on the SDS-PAGE. The major species of the smaller fragments of this protein were of 17 and 22 kDa. An immunoblot of the lysate of E. coli expressing 2C2 post induction with 1 mM of IPTG shows that the process of protein degradation has already initiated during the expression process in E. coli. Circular dichroism spectrum of 2C2 showed a spectrum of unfolded protein.

One third of AD patients have 2C2-specific IgE:
Serum IgE reactivity to nitrocellulose-blotted 2C2 was analysed using sera from 68 AD patients and 17 healthy individuals (Table III).

**Table III. Demographic, clinical and serological characterization of AD patients^{a}**

| | | **Age** | **Total IgE** | | | **Bacterial** | | **SAE IgE** | **anti-2C2IgE** | |
|---|---|---|---|---|---|---|---|---|---|---|
| **Patient** | **Sex** | **(years)** | **(kUl-1)** | **Symptoms** | **Allergen Sources** | **Infection** | **SCORAD** | **(RAST class)** | **Western blot** | **Dot blot** |
| 1 | f | 41 | 429 | AD | grass, mugwort, fungus, mite, animal fur | N | ND | ND | - | - |
| 3 | f | 58 | 1801 | AD, AA, RC | tree, grass, mugwort, mite, nut, fruit, vegetable | N | 11,8 | SEA (2), SEB (3) | (+) | (+) |
| 4 | m | 2 | 32,9 | AD | mite, nut, egg | N | ND | ND | - | - |
| 5 | f | 25 | 139 | AD, RC | tree, grass, vegetable | N | 16,8 | ND | - | - |
| 6 | f | 44 | 2795 | AD, RC | grass, mugwort, fruit | N | 31 | ND | (+) | (+) |
| 7 | f | 62 | 168 | AD, RA | tree, nut, vegetable | ND | 21,3 | ND | - | - |
| 8 | ND | ND | ND | ND | ND | ND | ND | ND | - | - |
| 9 | m | 31 | 10546 | AD | tree, grass, mold, fungus, mite, animal fur | Y | ND | ND | (+) | - |
| 11 | f | 43 | 1334 | AD, RC | mite, nickel | ND | 36,3 | ND | - | - |
| 12 | f | 8 | 22090 | AD, AA | tree, grass, mugwort, mold, mite, | N | ND | ND | + | + |
| | | | | | nut, animal fur, milk, fish | | | | | |
| 13 | ND | ND | ND | ND | ND | ND | ND | ND | - | - |
| 14 | f | 21 | 132 | AD, RA | grass, mold, fungus, mite, fruit | N | 32,5 | ND | - | - |
| 15 | m | 49 | 122 | AD | tree, fruit, animal fur, latex | N | ND | ND | + | - |
| 16 | f | 60 | 164 | AD, AA, RK | tree, mite | Y | ND | ND | - | - |
| 17 | m | 34 | 23900 | AD | grass, mold, mite, nut, | Y | ND | ND | ND | - |
| | | | | | vegetable, animal fur, egg, milk | | | | | |
| 18 | f | 25 | 6,58 | ND | mold, animal fur | ND | ND | ND | + | - |
| 19 | f | 3 | 281 | AD | nut, egg | N | 34,9 | ND | - | - |
| 20 | m | 40 | 448 | AD | tree, grass, mold, fungus, mite, animal fur | N | 41,2 | ND | - | - |
| 21 | m | 23 | 49,4 | RC | grass | N | ND | ND | - | - |
| 22 | f | 31 | 141 | AD, RC | fungus, fruit | Y | ND | ND | - | - |
| 23 | f | 29 | 28,3 | E | - | ND | ND | ND | - | - |
| 24 | f | 52 | 6748 | AD, RC, AA | tree, grass, mold, mite, nut, | ND | ND | ND | + | - |
| | | | | | vegetable, animal fur, egg, latex | | | | | |
| 25 | f | 17 | 2222 | AD, AA | nut, animal fur | Y | ND | ND | (+) | - |
| 26 | f | 24 | 61,8 | ND | tree, mugwort | ND | ND | ND | + | - |
| 27 | ND | ND | 140 | AD | egg | N | ND | ND | - | - |
| 28 | m | 29 | 39181 | AD | tree, grass, mugwort, mold, mite, animal fur | Y | ND | ND | + | - |
| 29 | f | 38 | 68,9 | AD, AA, RC | tree, grass, mugwort, mite, animal fur, drug | N | ND | ND | ND | - |
| 30 | f | 41 | 39181 | AD, RA | tree, grass, mugwort, fungus, | N | 26,9 | ND | + | - |
| | | | | | nut, vegetable, fruit, animal fur, latex | | | | | |
| 31 | m | 66 | 673 | ME | - | N | ND | ND | - | (+) |
| 32 | m | 34 | 130 | AD, RC | tree, grass, nut, animal fur, wasp | N | ND | ND | - | - |
| 33 | f | 30 | 21723 | AD | grass, mold, mite, animal fur | Y | ND | ND | ND | + |
| 34 | f | 43 | 105 | RA | tree, grass, mugwort, nut, vegetable, animal fur | | 29,1 | ND | - | ND |
| 35 | m | 33 | 18,1 | AD | wasp | N | ND | ND | - | - |
| 36 | m | 23 | 25328 | ND | tree, grass, mugwort, mold, mite, | ND | ND | ND | + | + |
| | | | | | nut, vegetable, animal fur, egg, milk, fish | | | | | |
| 37 | f | 21 | 9375 | AD, AA | tree, grass, mugwort, mite, | N | ND | ND | + | + |
| | | | | | nut, vegetable, animal fur, milk, fish | | | | | |
| 38 | m | 25 | 9265 | AD, RA | mite, nut, vegetable, egg, milk, fish | ND | ND | ND | + | + |
| 39 | f | 33 | 736 | AD, RC | animal fur | N | ND | ND | + | ND |
| 42 | f | 30 | 15,3 | AD | nd | ND | ND | ND | - | - |
| 43 | ND | ND | ND | ND | ND | ND | ND | ND | - | - |
| 44 | f | 42 | 692 | AD, RC, AA | tree, grass, animal fur | N | ND | ND | ND | - |
| 45 | m | 36 | 10,6 | AD | tree | N | 34,9 | ND | - | - |
| 46 | m | 65 | 95 | E | - | Y | ND | ND | ND | - |
| 48 | f | 69 | 793 | AD, U | tree, mite, nut, vegetable, fruit, animal fur, milk | ND | ND | ND | - | - |
| 49 | m | 67 | 3317 | AD | mite, vegetable | N | 15,8 | ND | + | + |
| 51 | f | 21 | 73,3 | AD | nd | ND | 68,3 | ND | - | - |
| 52 | f | 27 | 1122 | AD | tree, mite, nut, vegetable, fruit, animal fur | ND | 31,3 | ND | - | - |
| 53 | ND | ND | ND | ND | ND | ND | ND | ND | - | - |
| 54 | f | 54 | <2.0 | D | - | ND | ND | negative | - | - |
| 55 | m | 31 | 412 | AD, RC, AA | tree, grass, mugwort, nut, vegetable, | ND | ND | ND | - | + |
| | | | | | fruit, animal fur | | | | | |
| 56 | m | 45 | 166 | AD | grass, tree, mite, animal fur | ND | 13,5 | SEB (1) | - | - |
| 57 | ND | ND | ND | ND | ND | ND | ND | ND | - | - |
| 58 | f | 65 | 7290 | AD, AA, RC | grass, mite | ND | 15,8 | ND | - | (+) |
| 59 | m | 20 | 15290 | AD, AA | tree, grass, mugwort, mold, fungus, mite, nut | ND | 59 | SE(A+ B) (1) | (+) | - |
| | | | | | \vegetable, fruit, animal fur, egg, milk, fish, latex | | | | | |
| 60 | m | 35 | 25804 | AD, AA | tree, grass, mugwort, mold, fungus, mite, nut | ND | 42,1 | SEA (3), SEB (2) | (+) | + |
| | | | | | \vegetable, fruit, animal fur, egg, milk, fish, latex | | | | | |
| 61 | m | 38 | 6140 | AD, AA, AC | tree, grass, mugwort, mold, mite, | ND | 77 | SEA (1) | (+) | + |
| | | | | | nut, vegetable, fruit, animal fur, egg, milk | | | | | |
| 63 | f | 53 | 35,4 | AD, AA, RA | nut | N | ND | ND | - | - |
| 64 | f | 69 | 78,6 | AD | - | ND | ND | ND | - | - |
| 65 | m | 41 | 12254 | AD, AA, AC | tree, mugwort, mold, mite, nut, vegetable, | ND | 16,4 | SE(A+ B) (1) | (+) | + |
| | | | | | animal fur, fish | | | | | |
| MS | m | 38 | >2000 | AD, RC | tree, grass, mugwort, mold, fungus, mite, | ND | ND | ND | (+) | (+) |
| | | | | | nut, vegetable, fruit, animal fur, egg, milk, fish, latex | | | | | |
| MK | m | 41 | 3013 | AA, RC | nd | ND | ND | ND | (+) | - |
| T | m | 30 | >5000 | AD | tree, grass, mugwort, mold, mite, | ND | ND | ND | + | (+) |
| | | | | | nut, vegetable, animal fur, egg, milk, fish, latex | | | | | |
| S | f | 24 | >5000 | AD | tree, grass, mugwort, mold, mite, | ND | ND | ND | + | - |
| | | | | | nut, vegetable, animal fur, egg, milk, fish | | | | | |
| MD | m | 56 | 1720 | AD, RC | tree, grass, mugwort, mold, fungus, mite, | ND | ND | ND | (+) | (+) |
| | | | | | nut, vegetable, fruit, animal fur, egg, milk, fish, latex | | | | | |
| KY | f | 26 | 778 | AD, RC | grass, mite, fruit, animal fur, milk | ND | ND | ND | - | - |
| PH | m | 39 | 1060 | AD | tree, grass, mugwort, mold, mite, | ND | ND | SEA (0), SEB (0), | - | - |
| | | | | | nut, vegetable, animal fur | | | SEC (0), TSST-1 (1) | | |
| KM | m | 16 | 398 | AD | tree, grass, mugwort, mold, mite, | ND | ND | ND | - | - |
| | | | | | nut, vegetable, animal fur, egg, milk, fish | | | | | |
| SS | m | ND | 401 | ND | tree, grass, mugwort | ND | ND | ND | - | - |
| H | f | 27 | 67,7 | AD, RC | tree, grass, mite | ND | ND | ND | - | - |
| | | | | | | | | | | |

| | | | | | | | | | | |
|---|---|---|---|---|---|---|---|---|---|---|
| aAA, allergic asthma; AD, atopic dermatitis; AR, allergic rhinitis; f, female; kul-1, kilo units per liter; m, male; ND, not determined; N, no; RC, rhinoconjunctivitis; SAE, *Staphylococcus aureus* enterotoxin; SE, staphylococcal enterotoxin; TSST, toxic shock syndrome toxin; Y, yes; +, positive reaction; -, negative reaction; (+), weak positive reaction | | | | | | | | | | |

Of the AD patients, there were 34 female, 28 male and six were not determined. The patients were from a diverse age group, from two to 69 years old. About half of the patients suffered from other allergic diseases such as allergic asthma and allergic rhinitis, and were sensitized to diverse allergen sources.

Thirty four percent of the AD patients showed IgE binding to 2C2, while none of the non-allergic patients tested showed any IgE reaction (Figure 2). Elevated total serum IgE levels were often associated with IgE reactivity to 2C2, but were not a pre-requisite for IgE binding (Table III). Some patients with low total serum IgE (e.g., #15, 26, 39) had anti-2C2 IgE antibodies (Table III; Figure 2).

AD patients had elevated anti-2C2 IgG1 and IgG4 antibodies but not IgG2 antibody compared to healthy individuals:
The levels of specific anti-2C2 IgG antibody subtypes (IgG1, IgG2 and IgG4) in AD patients (n=38) in comparison to non-allergic individuals (n=7) were measured using an ELISA based assay. The median anti-2C2 IgG1 and IgG4 antibody levels were elevated in AD patients compared to healthy individuals (Figure 3). The median anti-2C2 IgG2 antibody level in healthy individuals was slightly higher than that of AD patients, but this difference was not statistically significant.

2C2 contains functional T-cell epitopes:
Peripheral blood mononuclear cells (PBMCs) were isolated from fresh blood of atopic dermatitis patients and non-allergic individuals, and stimulated with 2C2, Phl p 1 or S. aureus extract for seven days. The amount of T-cell proliferation upon antigen stimulation in comparison to medium controls was measured by the amount of thymidine incorporation (Table IV).

**Table IV. Proliferation of human PBMCs**

| | | 2C2 | | | Ph1 p1 | | | *S. aureus* extract | | |
|---|---|---|---|---|---|---|---|---|---|---|
| Patient | Maximum SI | 5.0 µg/w | 1.0 µg/w | 0.2 µg/w | 5.0 µg/w | 1.0 µg/w | 0.2 µg/w | 50.0 µg/w | 10.0 µg/w | 2.0 µg/w |
| T | 9.5 | 5.06 | 2.80 | 2.18 | 1.49 | 1.56 | 1.79 | 4.93 | 3.21 | 3.04 |
| S | 19.5 | 4.09 | 3.44 | 1.27 | 5.95 | 1.62 | 1.25 | 18.03 | 12.17 | 6.19 |
| PH | 2.8 | 1.89 | 2.05 | 2.14 | 0.29 | 0.82 | 0.66 | 3.62 | 0.66 | 1.07 |
| KY | 38.2 | 7.87 | 5.93 | 1.89 | 6.96 | 3.63 | 4.57 | 22.90 | 4.57 | 11.11 |
| SH | 2.2 | 2.23 | 2.63 | 1.85 | 2.05 | 1.27 | 1.50 | 4.14 | 2.14 | 3.20 |
| RV | 9.5 | 8.31 | 9.13 | 8.20 | 8.41 | 3.81 | 5.31 | 11.70 | 5.31 | 13.83 |

Among the six patients tested, patients T and S suffered from AD, and had anti-2C2 IgE based on the immunoblot results, patients PH and KY suffered from AD, but had no detectable IgE-reactivity to 2C2 on immunoblots, and patients SH and RV were healthy individuals (Figure 2). It was observed that 2C2 was able to stimulate T cell proliferation in a dose dependent manner in several patients (T, S, KY). The ability of 2C2 to elicit T cell proliferation was not dependent on the availability of specific anti-2C2 IgE, or the presence of AD in these patients. Phl p 1 was used as a control allergen in this assay, as this allergen has been previously identified to have intact T cell epitopes. The amount of T-cell proliferation to S. aureus extracts showed a great variation between the patients (Table IV).

2C2 is able to induce allergenic activity:
The ability of 2C2 to induce allergenic activity was studied using RBL cells expressing the human FcεRI receptor. The humanized RBL cells were loaded with anti-2C2 IgE antibodies from allergic (n=5) or a non-allergic individual (patient SH). As seen in Figure 4, one patient (S) displayed the highest amount of mediator release upon IgE-allergen cross linking on the humanized RBL cells, while another patient (MK) showed only modest amount of mediator release. The amount of mediator release in the other patients was below baseline values. The amount of mediator release by the humanized RBL cells was not correlated to the amount of anti-2C2 antibodies in the patients tested (Figure 2 and 4). Phl p 1 was used as a control protein in this assay, as it is a well characterized allergen.

Mice immunized with 2C2 generated anti-2C2 IgG1, IgG2a and IgE antibodies:
Mice immunized with recombinant 2C2 adsorbed to alum generated specific IgG1 and IgG2a antibodies as measured by an ELISA assay. The maximum titer of antibodies was produced from the third immunization onwards (Figure 5). Similarly, murine anti-2C2 IgE antibodies were generated at levels similar to IgG1 and IgG2a antibodies after the fourth immunization (Figure 5).

Lymphoproliferation of mice spleen cells after immunization with recombinant 2C2:
Spleens of mice (n=5) immunized subcutaneously with 2C2 were removed at the end of the immunization protocol, and fresh lymphocyte cells were isolated. These cells were stimulated with either 2C2, Phl p 1, S. aureus extract (SE) or concanavalin A (Con A) (Figure 6). It was observed that spleen lymphocyte cells from immunized mice showed higher proliferation when stimulated with 2C2, compared to SE or positive control Con A (Figure 6). Recombinant Phl p 1 was used as a control protein, and showed the lowest amount of lymphoproliferation.

RBL assay with anti-2C2 murine sera showed specific mediator release:
Sera from mice (n=5) before or after immunization with 2C2 were loaded on RBL cells, in the presence or absence of 2C2. We observed that the murine anti-2C2 IgE antibodies generated were able to cross-link on the RBL cells in presence of 2C2, resulting in mediator release (Figure 7). None of the negative controls tested showed appreciable amounts of mediator release (Figure 7).

Discussion:
In this study, the identification of an IgE binding protein from S. aureus, termed 2C2 is disclosed. 2C2 is a member of the fibrinogen binding protein A domain family, consisting of 565 amino acids, with a theoretical molecular weight of 65.8 kDa. Although this protein is not stable, spontaneously liberating into smaller protein fragments, it still maintains its allergenic and immunogenic properties.

One third of the 68 AD patients tested in this study had anti-2C2 IgE antibodies, while none of the healthy individuals showed any IgE reaction. In most cases, increased total serum IgE levels of the AD patients correlated with the presence of anti-2C2 IgE antibodies, although there were exceptions to this rule in several patients. 2C2 was capable of inducing the production of specific IgG1, IgG2a and IgE antibodies in mice immunized with this protein, and therefore is both an immunogenic and allergenic protein.

Apart from binding to IgE antibodies in AD patients, 2C2 was able to stimulate mediator release from RBL cells in both human and murine experiments. Additionally, 2C2 had functional T-cell epitopes, as seen by the active T-cell proliferation of both human and murine lymphocyte cells when stimulated by this protein. This induction of T cell proliferation also in normal persons and IgG antibodies shows that this protein can be used in general also for non-atopic persons. 2C2 was also able to cross-link specific anti-2C2 IgE antibodies, from human or murine sera, loaded on the appropriate RBL cells leading to mediator release, indirectly hinting that this protein is also capable of inducing immediate type skin reaction.

Serum of individuals suffering from AD contained higher amounts of anti-2C2 IgG1 and IgG4 antibodies, as compared to serum of non-allergic individuals, while there was no significant difference in the level of IgG2 antibodies between both patient groups. This finding shows that there is the presence of an active Th1 pathway in these individuals, in relation to their immune activity against 2C2.

2C2 belongs to the fibronectin-binding protein A domain family, which is a common protein domain in many prokaryotic organisms. However, when the primary amino acid sequence of 2C2 was compared to known fibronectin binding proteins from human pathogenic bacteria, proteins similar to 2C2 could not be found. Hence, it can be concluded that 2C2 is a unique fibronectin binding protein, with possibly low antibody cross-reactivity to known proteins, and therefore can be used as a good diagnostic marker for S. aureus infections. Additionally, as 2C2 is an IgE binding protein, it can also be used to assay for the presence of S. aureus specific IgE antibodies in human sera.

Accordingly, these results show that 2C2 is a suitable antigen for a S. aureus vaccine protein panel, as it is a soluble antigen, which displays strong immunogenic and antigenic properties.

References:
Baba et al., Lancet 359 (2002):1819-1827.
Diep et al., Lancet 367 (2006):731-739.
Gill et al., J Bacteriol 187 (2005):2426-2438.
Herron-Olson et al., PLoS One 2 (2007):e1120.
Holden et al., Proc Natl Acad Sci U S A 101 (2004):9786-9791.
Kuroda et al., Lancet 357 (2001):1225-1240.
Laemmli, Nature 227 (1970):680-685.
Linhart et al., J Immunol 178 (2007):3924-3931.
Ohta et al., DNA Res 11 (2004):51-56.
Schaffer et al., Int J Antimic. Agents 32 Suppl 1(2008):S71-78.
Towbin et al., Proc Natl Acad Sci U S A 76 (1979):4350-4354.

## Claims

1. : Method for analysing a sample for identifying infections with S. aureus wherein the presence or absence of a protein with an amino acid sequence according to SEQ.ID.NO.1 or a naturally occurring fragment or variant thereof or the presence or absence of a nucleic acid encoding said protein, fragment or variant in the sample is tested.

2. : Method according to claim 1, **characterised in that** the sample is a sample from a human patient.

3. : Method according to claim 1 or 2, **characterised in that** the method tests the presence or absence of a nucleic acid encoding said protein, fragment or variant by polymerase chain reaction PCR).

4. : Method for detecting antibody molecules being specific for a protein with an amino acid sequence according to SEQ.ID.NO.1 or antibody binding fragments or variants thereof in an antibody containing sample, **characterised in that** the sample is contacted with a protein with an amino acid sequence according to SEQ.ID.NO.1 or an antibody binding fragment or variant thereof and whereafter it is analysed whether any of the antibody molecules have bound to the protein with an amino acid sequence according to SEQ.ID.NO.1 or the antibody binding fragment or variant thereof.

5. : Method for detecting IgE molecules being specific for a protein with an amino acid sequence according to SEQ.ID.NO.1 or IgE binding fragments or variants thereof in an IgE containing sample, **characterised in that** the sample is contacted with a protein with an amino acid sequence according to SEQ.ID.NO.1 or an IgE binding fragment or variant thereof and whereafter it is analysed whether any of the IgE molecules have bound to the protein with an amino acid sequence according to SEQ.ID.NO.1 or the IgE binding fragment or variant thereof.

6. : Method according to claim 4 or 5, **characterised in that** the protein with an amino acid sequence according to SEQ.ID.NO.1 or the antibody binding fragment or variant thereof is contacted with the antibody containing sample in a form bound to a solid surface; and that the antibody containing sample is separated from this solid surface after the contact, whereafter the analysis whether any of the antibody molecules have bound to the protein with an amino acid sequence according to SEQ.ID.NO.1 or the antibody binding fragment or variant thereof is performed on the solid surface in a form separated from the sample.

7. : Method according to any one of claims 1 to 6, **characterised in that** the method is an enzyme-linked immunosorbent assay (ELISA).

8. : Naturally occurring fragment of a protein with an amino acid sequence according to SEQ.ID.NO.1 or a naturally occurring variant thereof in isolated form.

9. : Protein fragment according to claim 8, **characterised in that** said fragment is an IgE binding fragment.

10. : Protein fragment according to claim 9, **characterised in that** said fragment is a fragment having an apparent molecular weight in SDS page electrophoresis of 17 kDa or a fragment having an apparent molecular weight in SDS page electrophoresis of 22 kDa.

11. : Pharmaceutical composition comprising a protein with an amino acid sequence according to SEQ.ID.NO.1 or a naturally occurring fragment or variant thereof and a pharmaceutically acceptable carrier.

12. : Vaccine comprising a protein with an amino acid sequence according to SEQ.ID.NO.1 or a naturally occurring fragment or variant thereof and a pharmaceutically acceptable carrier.

13. : Preparation containing isolated antibodies against a protein with an amino acid sequence according to SEQ.ID.NO.1 or a naturally occurring fragment or variant thereof.

14. : Nucleic acid encoding a protein with an amino acid sequence according to SEQ.ID.NO.1 or a naturally occurring fragment or variant thereof, said nucleic acid being free of other open reading frames for proteins being naturally encoded adjacently to SEQ.ID.NO.2 in S. aureus.

15. : Expression vector comprising a nucleic acid according to claim 14.


SEQUENCE LISTING

<110>  Biomay AG

<120>  S. AUREUS ALLERGEN

<130>  R 54912

<160>  2

<170>  PatentIn version 3.4

<210>  1
<211>  565
<212>  PRT
<213>  Staphylococcus aureus

<400>  1

Met Ala Tyr Asp Gly Leu Phe Thr Lys Lys Met Val Glu Ser Leu Gln
1               5                   10                  15

Phe Leu Thr Thr Gly Arg Val His Lys Ile Asn Gln Pro Asp Asn Asp
            20                  25                  30

Thr Ile Leu Met Val Val Arg Gln Asn Arg Gln Asn His Gln Leu Leu
        35                  40                  45

Leu Ser Ile His Pro Asn Phe Ser Arg Leu Gln Leu Thr Thr Lys Lys
    50                  55                  60

Tyr Asp Asn Pro Phe Asn Pro Pro Met Phe Ala Arg Val Phe Arg Lys
65                  70                  75                  80

His Leu Glu Gly Gly Ile Ile Glu Ser Ile Lys Gln Ile Gly Asn Asp
                85                  90                  95

Arg Arg Ile Glu Ile Asp Ile Lys Ser Lys Asp Glu Ile Gly Asp Thr
            100                 105                 110

Ile Tyr Arg Thr Val Ile Leu Glu Ile Met Gly Lys His Ser Asn Leu
        115                 120                 125

Ile Leu Val Asp Glu Asn Arg Lys Ile Ile Glu Gly Phe Lys His Leu
    130                 135                 140

Thr Pro Asn Thr Asn His Tyr Arg Thr Val Met Pro Gly Phe Asn Tyr
145                 150                 155                 160

Glu Ala Pro Pro Thr Gln His Lys Ile Asn Leu Tyr Asp Ile Thr Gly
                165                 170                 175

Ala Glu Val Leu Lys Tyr Ile Asp Phe Asn Ala Gly Asn Ile Ala Lys
            180                 185                 190

Gln Leu Leu Asn Gln Phe Glu Gly Phe Ser Pro Leu Ile Thr Asn Glu
        195                 200                 205

Ile Val Ser Arg Arg Gln Phe Met Thr Ser Ser Thr Leu Pro Glu Ala
    210                 215                 220

Phe Asp Glu Val Met Ala Glu Thr Lys Leu Pro Pro Thr Pro Ile Phe
225                 230                 235                 240

His Lys Asn His Glu Thr Gly Lys Glu Asp Phe Tyr Phe Ile Lys Leu
                245                 250                 255

Asn Gln Phe Asn Asp Asp Thr Val Thr Tyr Asp Ser Leu Asn Asp Leu
            260                 265                 270

Leu Asp Arg Phe Tyr Asp Ala Arg Gly Glu Arg Glu Arg Val Lys Gln
        275                 280                 285

Arg Ala Asn Asp Leu Val Arg Phe Val Gln Gln Gln Leu His Lys Tyr
    290                 295                 300

Gln Asn Lys Leu Ala Lys Leu Ile Glu Glu Tyr Glu Gln Ser Lys Asn
305                 310                 315                 320

Lys Asp Thr Glu Gln Leu Tyr Gly Glu Leu Ile Thr Ala Asn Ile Tyr
                325                 330                 335

Arg Ile Lys Gln Gly Asp Lys Glu Val Thr Ala Leu Asn Tyr Tyr Thr
            340                 345                 350

Asn Glu Glu Val Val Ile Pro Leu Asn Pro Thr Lys Ser Pro Ser Ala
        355                 360                 365

Asn Ala Gln Tyr Tyr Tyr Lys Gln Tyr Asn Arg Met Lys Thr Arg Glu
    370                 375                 380

Arg Glu Leu Gln His Gln Ile Gln Leu Thr Lys Asp Asn Ile Asp Tyr
385                 390                 395                 400

Phe Ser Thr Ile Glu Gln Gln Leu His His Ile Ser Val His Asp Ile
                405                 410                 415

Asp Glu Ile Arg Asp Glu Leu Ala Glu Gln Gly Phe Met Lys Gln Arg
            420                 425                 430

Lys Asn Gln Thr Lys Lys Lys Lys Ala Gln Ile Gln Leu Gln His Tyr
        435                 440                 445

Val Ser Thr Asp Gly Asp Asp Ile Tyr Val Gly Lys Asn Asn Lys Gln
    450                 455                 460

Asn Asp Tyr Leu Thr Asn Lys Lys Ala Lys Lys Thr His Thr Trp Leu
465                 470                 475                 480

His Thr Lys Asp Ile Pro Gly Ser His Val Val Ile Phe Asn Asp Ala

485                 490                 495

Pro Ser Asp Thr Thr Ile Lys Glu Ala Ala Met Leu Ala Gly Tyr Phe
            500                 505                 510

Ser Lys Ala Gly Asn Ser Gly Gln Ile Pro Val Asp Tyr Thr Leu Ile
        515                 520                 525

Lys Asn Val His Lys Pro Ser Gly Ala Lys Pro Gly Phe Val Thr Tyr
    530                 535                 540

Asp Asn Gln Lys Thr Leu Tyr Ala Thr Pro Asp Tyr Glu Leu Ile Gln
545                 550                 555                 560

Lys Met Lys Gln Ser
                565

<210>  2
<211>  1698
<212>  DNA
<213>  Staphylococcus aureus

<400>  2
atggcttatg atggcttatt tacaaagaaa atggttgagt ctctacaatt tttaacaaca      60

ggacgtgttc acaaaatcaa tcaacctgat aatgacacga tactaatggt tgtacgtcaa     120

aatagacaaa accatcaatt gttattgtca atccatccaa acttttcaag attacaattg     180

actactaaaa aatatgataa tccatttaat ccacccatgt ttgcgcgtgt ttttagaaaa     240

cacttagaag gtggtattat cgaatcgatt aagcaaattg gtaatgatcg tcgcattgaa     300

atcgatataa agagtaaaga tgaaattggc gatactattt accgcactgt catccttgag     360

attatgggta aacatagtaa cttaatttta gtagatgaaa atcgcaaaat aattgaagga     420

tttaaacact taacaccaaa tacgaatcac tatcgtacag taatgccagg atttaattat     480

gaagcaccac ctactcagca caaaataaat ctgtatgata ttacaggtgc agaggtgttg     540

aaatatatcg attttaacgc aggtaatatt gctaaacaat tattgaatca gtttgaagga     600

tttagccctt taattacgaa tgaaatcgtt agtcgtcgtc aatttatgac ttcatcaaca     660

ttaccagaag catttgacga agtaatggca gaaaccaagt taccacctac tcctattttt     720

cataaaaatc atgaaacagg taaagaggat ttctatttta taaagttaaa tcaatttaat     780

gatgatacag ttacatacga ttcattaaat gatttgcttg atcgttttta tgatgcgcgt     840

ggcgaacgtg aacgcgttaa acaacgtgcg aatgatttag ttcgatttgt tcaacagcag     900

ttgcacaaat atcaaaataa attagcgaag ttgattgaag aatatgagca gtctaaaaat     960

aaagatactg aacagttata tggtgaattg atcactgcta atatatatcg aattaagcaa    1020

ggcgataaag aagtgacggc attgaattat tatacgaatg aagaagttgt cattccttta    1080

aatcctacaa aatccccatc agcaaatgct caatattatt ataaacaata taatcgtatg    1140

aaaacgagag aacgtgaatt acaacatcaa attcaattga cgaaagacaa tatagattat    1200

ttttcaacaa tcgaacaaca attacatcat atttctgtcc atgacattga tgaaattaga    1260

gatgaattag cagaacaagg ctttatgaaa cagcgtaaaa atcaaactaa gaaaaagaaa 1320
gcgcagattc aattacaaca ttatgtatca actgatggcg acgatatata tgttggtaag 1380
aataacaagc aaaatgatta tttaacaaat aaaaaagcta aaaaaactca cacatggtta 1440
cacacaaaag atattcctgg ttcacatgtc gttatattta atgatgcacc aagtgatacg 1500
acaatcaagg aagcggctat gttagcagga tacttttcaa aagctggtaa ttctggacaa 1560
atacctgttg attatacatt aattaaaaat gtgcataaac catcaggtgc aaagcctggg 1620
tttgtaacat atgacaatca aaaaactttg tatgctacac ctgattatga acttattcaa 1680
aaaatgaaac aatcataa 1698